## Europäisches Patentamt

## European Patent Office

## Office européen des brevets

(11) Publication number: **0 091 313**
**B1**

(12) **EUROPEAN PATENT SPECIFICATION**

(45) Date of publication of patent specification: **05.09.90**

(51) Int. Cl.⁵: **A 61 K 9/06,** A 61 K 9/10, A 61 K 31/02

(21) Application number: **83301908.6**

(22) Date of filing: **05.04.83**

(54) **Perfluorohydrocarbons as vehicles for administering drugs.**

(30) Priority: **05.04.82 US 365539**

(43) Date of publication of application:
**12.10.83 Bulletin 83/41**

(45) Publication of the grant of the patent:
**05.09.90 Bulletin 90/36**

(84) Designated Contracting States:
**AT BE CH DE FR GB IT LI LU NL SE**

(56) References cited:
**EP-A-0 089 815**
**JP-A-8 221 312**
**US-A-3 219 533**
**US-A-4 352 789**

(73) Proprietor: **ALCON LABORATORIES INC**
**6201 South Freeway P.O. Box 1959**
**Fort Worth Texas 76101 (US)**

(72) Inventor: **York, Billie Murray, Jr.**
**3828 Winifred**
**Fort Worth Texas (US)**

(74) Representative: **Allard, Susan Joyce et al**
**BOULT, WADE & TENNANT, 27 Furnival Street**
**London EC4A 1PQ (GB)**

**Description**

This invention relates to compositions for administration of drugs, and, more particularly, this invention relates to compositions containing perfluorohydrocarbon vehicles for drugs for ocular or dermatological application.

Many therapeutic drugs have the disadvantage of being relatively unstable in an aqueous medium. Examples of this category of drugs include cephaloridine, cefamandole, cefamandole nafate, cefazolin, cefoxitin, cephacetrile sodium, cephalexin, cefoperazone sodium, cephaloglycin, cephalosporin C, cephalothin, nafcillin sodium, cephamycins, cephapirin sodium, cepharidine, penicillin BT, penicillin N, penicillin O, phenethicillin potassium, pivampicillin, amoxicillin, ampicillin, thienamycin, moxalactam, and cefatoxin.

Many therapeutic drugs are relatively water-insoluble. Examples of this category of drugs include vidarabine, prednisoline, prednisolone acetate, hydrocortisone, hydrocortisone acetate, hydrocortisone valerate, fluorometholone, fluocinolone acetonide, triamcinolone acetonide, dexamethasone, dexamethasone acetate, indomethacin, ibuprofen, and oxyphenbutazone.

Typically, oils or ointments have been used as vehicles for therapeutic drugs which are not stable in an aqueous medium or are relatively insoluble in an aqueous medium. These vehicles often are messy, leave a greasy afterfeel, and are particularly undesirable from a patient's perspective for topical application to the eye. Further, because of their nature, oils and ointments do not readily provide a metered dose to the area of application.

A need exists, therefore, for an improved vehicle for the topical ocular or dermatological application of therapeutic drugs which are water-unstable, or relatively insoluble in an aqueous medium.

Accordingly, the present invention provides a composition for the administration of a metered dose of ocular or dermatological drugs which are unstable or insoluble in water which comprises a therapeutically effective amount of an ocular or dermatological drug which is unstable or insoluble in water contained in a vehicle which comprises a perfluorohydrocarbon having a vapor pressure at 37°C of from 133.3 to 2133.2 Pa (1 to 16 mm Hg).

The perfluorohydrocarbons useful as vehicles are perfluorocycloalkanes, perfluoroalkanes, and perfluorotrialkylamines such as perfluorotripropylamine, perfluorotributylamine, perfluorotripentylamine, and mixtures thereof having a vapor pressure 133.3 to 2133.2 Pa (1 to 16 mm Hg). Although the perfluorohydrocarbon may be in the form of an aqueous microemulsion, in the preferred form of the invention, the perfluorohydrocarbon may form the entire vehicle. Specific examples of perfluorohydrocarbons include perfluorodecalin, $C_{10}F_{18}$ which has a vapor pressure of 1693.2 Pa (12.7 mm Hg) at 37°C, perfluorotributylamine, $N(CF_2CF_2CF_2CF_3)_3$ which has a vapor pressure of 152 Pa (1.14 mm Hg) at 37°C; perfluoromethyldecalin which has a vapor pressure of 639.9 Pa (4.8 mm Hg) at 37°C; perfluorocyclohexyldiethylamine which has a vapor pressure of 1159.9 pa (8.7 mm Hg) at 37°C; perfluoroisopentylpyran which has a vapor pressure of 1319.9 Pa (9.9 mm Hg) at 37°C; perfluorodibutylmethylamine which has a vapor pressure of 2133.2 Pa (16.0 mm Hg) at 37°C; and perfluorobutyltetrahydrofuran. These compounds are known for their use in blood substitutes, are nontoxic, are approved for human systemic use in various countries including the United States, generally are transparent and colorless and leave no stains, are relatively inert, and are easily prepared from commercially available chemicals, or are commercially available.

The inert nature and the vapour pressure of the fluorohydrocarbons of the invention are an important aspect of the invention. The inert nature of perfluorohydrocarbons permits them to be used by the patient with little or no toxic danger. The vapor pressure of the perfluorohydrocarbons of the invention permits the perfluorohydrocarbon vehicle to vaporize to provide a metered dose of a therapeutic drug, yet in ocular use not cause discomfort to the eye as would be the case with an ointment or cream. Further, the vapor pressure of the perfluorohydrocarbons of the invention do not create messy conditions with nonaqueous ocular or dermatological use which are normally associated with drugs used with nonaqueous vehicles.

The perfluorohydrocarbons used in the invention are used as a vehicle for therapeutic drugs which are not stable or soluble in water. Where therapeutic drugs are not compatible with aqueous vehicles, the drugs may be suspended or emulsified in the perfluorohydrocarbons. Vaporization of the perfluorohydrocarbons meters the dosage of the therapeutic drug suspended in the perfluorohydrocarbons.

The effective amount of perfluorohydrocarbons used in the invention will depend upon whether the use is ocular or dermatological, the metered dose desired, and the selection as well as strength of the drug to be applied. Generally, an effective amount of perfluorohydrocarbons is provided for ocular use when the ophthalmic composition contains between about 98 percent and about 99.99 percent by weight perfluorohydrocarbons. For dermatological use, generally an effective amount of perfluorohydrocarbons is provided when the composition contains between about 97 percent and about 99.99 percent by weight perfluorohydrocarbons.

The present invention also includes within its scope a process for the preparation of a composition for the administration of a metered dose of ocular or dermatological drugs which are unstable or insoluble in water which comprises mixing a therapeutically effective amount of an ocular or dermatological drug which is unstable or insoluble in water with a vehicle which comprises a perfluorohydrocarbon having a vapour pressure at 37°C of from 133.3 to 2133.2 Pa (1 to 16 mm Hg).

The following examples typify the manner by which the present invention can be practiced.

Example I

Sodium cefamandole powder (1.0 g supplied by Eli Lilly Co.) is added to perfluorotributylamine (200 cc supplied by Pfaltz and Bauer), is manually or mechanically shaken and a uniform 0.5 percent w/v suspension results. On standing, the sodium cefamandole will float to the top of the vehicle; however manual shaking will readily resuspend the sodium cefamandole antibiotic uniformly.

Example II

Indomethacin powder (1.0 g) is added to perfluorotributylamine (200 cc supplied by Pfaltz and Bauer) and is manually shaken to yield a uniform 0.5 percent w/v suspension. On standing, the indomethacin will float to the top of the vehicle; however, manual shaking will readily resuspend the indomethacin antiinflammatory agent uniformly.

In vitro evaluation of stability

Sodium cefamandole is stable only for hours at room temperature in aqueous solution. In the perfluorotributylamine vehicle at room temperature, however, sodium cefamandole was found to be chemically stable for five months. Further, after the five months the sodium cefamandole lost no antibacterial activity when evaluated in standard microbiological *in vitro* assays.

In vivo evaluation of formulation

In a study involving eight groups of six albino rabbits (12 eyes per group), sodium cefamandole as formulated in Example I of the invention was found to be as effective as freshly prepared 0.5 percent w/v aqueous sodium cefamandol in eradicating ocular infection and more effective than 0.5% w/v chloramphenicol ophthalmic solution. U.S.P., which is a commercially recognized occular drug product.

The study was conducted as follows:

a) Twenty-four albino rabbits were inoculated in their corneas (48 eyes) with *Staphylococcus aureus*. At random, six of these inoculated rabbits became treatment Group A, six became treatment Group B, six became treatment Group C, and six became the untreated control Group D.

b) Twenty-four albino rabbits were inoculated in their corneas (48 eyes) with *Streptococcus pneumoniae* (formerly *Diplococcus pneumoniae*). At random, six of these inoculated rabbits became treatment Group E, six became treatment Group F, six became treatment Group G, and six untreated became the control Group H.

Groups A and E were treated with an Example I formulation (0.5% w/v) of the sodium cefamandole in perfluorotributylamine. Groups B and F were treated with freshly prepared (0.5% w/v) aqueous sodium cefamandole. Groups C and G were treated with a solution of (0.5% w/v) chloramphenicol opthalmic solution, U.S.P.

The study was conducted as follows:

Group A was treated with one drop (100 ul) of 0.5% w/v sodium cefamandole in perfluorotributylamine one hour post inoculation and then once an hour thereafter for a total of nine doses. Then the eyes were graded for signs of infection 24 hours thereafter. At that time the eyes were examined with a slit lamp microscope. All eyes were found without signs of infection and all eyes (12) were found to be normal.

Group B was treated with one drop (100 ul) of freshly prepared 0.5% w/v aqueous sodium cefamandole one hour post inoculation and then once an hour thereafter for a total of nine doses. The ocular observations were as that stated for Group A. All eyes were found without signs of infection at 24 hours. All eyes (12) were found to be normal.

Group C was treated with drops (100 ul each) of 0.5% w/v chloramphenicol ophthalmic solution, U.S.P., as in the protocol of Groups A and B. In this case, the eyes showed signs of infection particularly at 24 hours. Eleven of twelve eyes showed signs of infection.

Group D was untreated and all eyes (12) showed signs of infection at 24 hours.

Group E was treated with one drop (100 ul) of 0.5% w/v sodium cefamandole in perfluorotributylamine one hour post inoculation and then once an hour thereafter for a total of nine doses. Then the eyes were graded for signs of infection at 24, 48 and 72 hours with gross and slit lamp microscopic observation. Most eyes (11/12) were found without signs of infection and normal at 72 hours.

Group F was treated with drops (100 ul each) of 0.5% w/v of sodium cefamandole as in the protocol of Group E. As in the case of Group E most eyes (10/12) were found to be without infection and normal at 72 hours.

Group G was treated with drops (100 ul each) of 0.5% chloramphenicol ophthalmic solution, U.S.P., as in the protocol of Group E. In this case, most eyes (10/12) at 72 hours showed signs of infection.

Group H was untreated and all eyes (12) showed signs of infection at 72 hours.

The conclusion is that sodium cefamandole in trifluorotributylamine is as effective as soluble sodium cefamandole in aqueous vehicle except that the cefamandole is stable for substantially longer periods than

3

water-solubilized sodium cefamandole. In the perfluorotributylamine, the B-lactam ring of cefamandole is not subject to either acid-catalyzed or base-promoted hydrolysis because water is not present to react.

In vivo ocular tolerance of the perfluorotributylamine vehicle

In one day, multiple (12) topical ocular dose (50 ul drops) regiments of neat perfluorotributylamine vehicle were applied to albino rabbit eyes. There were no detectable untoward ocular side-effects.

## Claims

1. A composition for the administration of a metered dose of ocular or dermatological drugs which comprises a therapeutically effective amount of an ocular or dermatalogical drug which is unstable or insoluble in water contained in a vehicle which comprises a perfluorohydrocarbon having a vapor pressure at 37°C of from 133.3 to 2133.2 Pa (1 to 16 mm Hg).

2. A composition as claimed in claim 1 wherein the perfluorohydrocarbon vehicle comprises a perfluoroalkane.

3. A composition as claimed in claim 1 wherein the perfluorohydrocarbon vehicle comprises a perfluorocycloalkane.

4. A composition as claimed in claim 1 wherein the perfluorohydrocarbon vehicle comprises a perfluorotrialkylamine having from 9 to 15 carbon atoms.

5. A composition as claimed in claim 1 wherein the perfluorohydrocarbon vehicle comprises perfluorodecalin.

6. A composition as claimed in claim 1 wherein the perfluorohydrocarbon vehicle comprises perfluorotributylamine.

7. A composition as claimed in claim 1 wherein the perfluorohydrocarbon vehicle comprises perfluoromethyldecalin.

8. A composition as claimed in claim 1 wherein the perfluorohydrocarbon vehicle comprises perfluorocyclohexyldiethylamine.

9. A composition as claimed in claim 1 wherein the perfluorohydrocarbon vehicle comprises perfluoroisopentylpyran.

10. A composition as claimed in claim 1 wherein the perfluorohydrocarbon vehicle comprises perfluorodibutylmethylamine.

11. A composition as claimed in claim 1 wherein the perfluorohydrocarbon vehicle comprises perfluorobutyltetrahydrofuran.

12. A composition as claimed in any one of the preceding claims which is an opthalmic composition and contains from 98 to 99.99 percent by weight of the perfluorohydrocarbon.

13. A composition as claimed in any one of the preceding claims which is a dermatological composition and contains from 97 to 99.99 percent by weight of the perfluorohydrocarbon.

14. A composition as claimed in any one of the preceding claims which is the form of a solution, suspension or micro-emulsion.

15. A composition as claimed in any one of the preceding claims wherein the therapeutic drug is cefamandole or indomethacin.

16. A process for the preparation of a composition for the administration of a metered dose of ocular or dermatological drugs which comprises mixing a therapeutically effective amount of an ocular or dermatological drug which is unstable or insoluble in water with a vehicle which comprises a perfluorohydrocarbon having a vapour pressure at 37°C of from 133.3 to 2133.2 Pa (1 to 16 mm Hg).

17. A process as claimed in claim 16 wherein the composition is a solution, a suspension, or a micro-emulsion.

18. A process as claimed in claim 16 or claim 17 wherein the therapeutic drug is cefamandole, or indomethacin.

19. A method of stabilizing a water-instable or water-insoluble drug for ocular or dermatological delivery, which method comprises forming a solution, suspension or micro-emulsion of the ocular or dermatological drug in a vehicle which comprises a perfluorohydrocarbon having a vapour pressure at 37°C of from 133.3 to 2133.2 Pa (1 to 16 mm Hg), the vehicle being present in an amount of from 98 to 99.99 wt.% when the drug is an ocular drug and from 97 to 99.99 wt.% when the drug is a dermatological drug, both percentages being with respect to the solution, suspension or microemulsion.

## Patentansprüche

1. Zusammensetzung zur Verabreichung einer zugemessenen Dosis von okularen oder dermatologischen Arzneimitteln, umfassend eine therapeutisch wirksame Menge eines okularen oder dermatologischen Arzneimittels, das in Wasser instabil oder unlöslich ist, enthalten in einem Vehikel, das einen Perfluorkohlenwasserstoff mit einem Dampfdruck von 133,3 bis 2133,2 Pa (1 bis 16 mm Hg) bei 37°C enthält.

2. Zusammensetzung nach Anspruch 1, worin das Perfluorkohlenwasserstoff-Vehikel ein Perfluoralkan enthält.

3. Zusammensetzung nach Anspruch 1, worin das Perfluorkohlenwasserstoff-Vehikel ein Perfluorcycloalkan enthält.

4. Zusammensetzung nach Anspruch 1, worin das Perfluorkohlenwasserstoff-Vehikel ein Perfluortrialkylamin mit 9 bis 15 Kohlenstoffatomen enthält.

5. Zusammensetzung nach Anspruch 1, worin das Perfluorkohlenwasserstoff-Vehikel Perfluordecalin enthält.

6. Zusammensetzung nach Anspruch 1, worin das Perfluorkohlenwasserstoff-Vehikel Perfluortributylamin enthält.

7. Zusammensetzung nach Anspruch 1, worin das Perfluorkohlenwasserstoff-Vehikel Perfluormethyldecalin enthält.

8. Zusammensetzung nach Anspruch 1, worin das Perfluorkohlenwasserstoff-Vehikel Perfluorcyclohexyldiethylamin enthält.

9. Zusammensetzung nach Anspruch 1, worin das Perfluorkohlenwasserstoff-Vehikel Perfluorisopentylpyran enthält.

10. Zusammensetzung nach Anspruch 1, worin das Perfluorkohlenwasserstoff-Vehikel Perfluordibutylamin enthält.

11. Zusammensetzung nach Anspruch 1, worin das Perfluorkohlenwasserstoff-Vehikel Perfluorbutyltetrahydrofuran enthält.

12. Zusammensetzung nach einem der vorhergehenden Ansprüche, die eine ophthalmische Zusammensetzung ist und von 98 bis 99,99 Gew.-% des Perfluorkohlenwasserstoffs enthält.

13. Zusammensetzung nach einem der vorhergehenden Ansprüche, die eine dermatologische Zusammensetzung ist und von 97 bis 99,99 Gew.-% des Perfluorkohlenwasserstoffs enthält.

14. Zusammensetzung nach einem der vorhergehenden Ansprüche, die in Form einer Lösung, Suspension oder Mikroemulsion ist.

15. Zusammensetzung nach einem der vorhergehenden Ansprüche, worin das therapeutische Arzneimittel Cephamandol oder Indomethacin ist.

16. Verfahren zur Herstellung einer Zusammensetzung zur Verabreichung einer zugemessenen Dosis von okularen oder dermatologischen Arzneimitteln, umfassend das Vermischen einer therapeutisch wirksamen Menge eines okularen oder dermatologischen Arzneimittels, das in Wasser instabil oder unlöslich ist, mit einem Vehikel, das einen Perfluorkohlenwasserstoff mit einem Dampfdruck von 133,3 bis 2133,2 Pa (1 bis 16 mm Hg) bei 37°C enthält.

17. Verfahren nach Anspruch 16, worin die Zusammensetzung eine Lösung, Suspension oder eine Mikroemulsion ist.

18. Verfahren nach Anspruch 16 oder 17, worin das therapeutische Arzneimittel Cephamandol oder Indomethacin ist.

19. Verfahren zur Stabilisierung eines in Wasser instabilen oder in Wasser unlöslichen Arzneimittels zur okularen oder dermatologischen Verabreichung, wobei das Verfahren die Herstellung einer Lösung, Suspension oder Mikroemulsion des okularen oder dermatologischen Arzneimittels in einem Vehikel umfaßt, das einen Perfluorkohlenwasserstoff mit einem Dampfdruck von 133,3 bis 2133,2 Pa (1 bis 16 mm Hg) bei 37°C enthält, wobei das Vehikel in einer Menge von 98 bis 99,99 Gew.-% vorhanden ist, wenn das Arzneimittel ein okulares Arzneimittel ist, und von 97 bis 99,99 Gew.-%, wenn das Arzneimittel ein dermatologisches Arzneimittel ist, wobei sich beide Prozentangaben auf die Lösung, Suspension oder Mikroemulsion beziehen.

**Revendications**

1. Composition pour l'administration d'une dose mesurée de médicaments oculaires ou dermatologiques qui comprend une quantité active du point de vue thérapeutique d'un médicament oculaire ou dermatologique qui est instable ou insoluble dans l'eau contenue dans un véhicule qui comprend un perfluorohydrocarbure présentant une pression de vapeur à 37°C comprise entre 133,3 et 2133,2 Pa (1 à 16 mm Hg).

2. Composition selon la revendication 1 dans laquelle le véhicule de perfluorohydrocarbure comprend un perfluoroalcane.

3. Composition selon la revendication 1 dans laquelle le véhicule de perfluorohydrocarbure comprend un perfluorocycloalcane.

4. Composition selon la revendication 1 dans laquelle le véhicule de perfluorohydrocarbure comprend une perfluorotrialkylamine comportant de 9 à 15 atomes de carbone.

5. Composition selon la revendication 1 dans laquelle le véhicule de perfluorohydrocarbure comprend une perfluorodécaline.

6. Composition selon la revendication 1 dans laquelle le véhicule de perfluorohydrocarbure comprend une perfluorotributylamine.

7. Composition selon la revendication 1 dans laquelle le véhicule de perfluorohydrocarbure comprend une perfluorométhyldécaline.

8. Composition selon la revendication 1 dans laquelle le véhicule de perfluorohydrocarbure comprend une perfluorocyclohexyldiéthylamine.

9. Composition selon la revendication 1 dans laquelle le véhicule de perfluorohydrocarbure comprend un perfluoroisopentylpyrane.

10. Composition selon la revendication 1 dans laquelle le véhicule de perfluorohydrocarbure comprend une perfluorodibutylméthylamine.

11. Composition selon la revendication 1 dans laquelle le véhicule de perfluorohydrocarbure comprend un perfluorobutyltétrahydrofurane.

12. Composition selon l'une quelconque des revendications précédentes qui est une composition ophtalmique et contient de 98 à 99,99 pour cent en poids du perfluorohydrocarbure.

13. Composition selon l'une quelconque des revendications précédentes qui est une composition dermatologique et contient de 97 à 99,99 pour cent en poids du perfluorohydrocarbure.

14. Composition selon l'une quelconque des revendications précédentes qui est sous la forme d'une solution, suspension ou micro-émulsion.

15. Composition selon l'une quelconque des revendications précédentes dans laquelle le médicament thérapeutique est du céfamandole ou de l'indométhacine.

16. Procédé pour la préparation d'une composition pour l'administration d'une dose mesurée de médicaments oculaires ou dermatologiques qui consiste à mélanger une quantité active du point de vue thérapeutique d'un médicament oculaire ou dermatologique qui est instable ou insoluble dans l'eau avec un véhicule qui comprend un perfluorohydrocarbure présentant une pression de vapeur à 37°C comprise entre 133,3 et 2133,2 Pa (1 à 16 mm Hg).

17. Procédé selon la revendication 16 dans lequel la composition est une solution, une suspension, ou une microémulsion.

18. Procédé selon la revendication 16 ou 17 dans lequel le médicament thérapeutique est du céfamandole ou de l'indométhacine.

19. Procédé de stabilisation d'un médicament instable dans l'eau ou insoluble dans l'eau pour la fourniture d'un produit oculaire ou dermatologique, lequel procédé consiste à former une solution, suspension ou micro-émulsion du médicament oculaire ou dermatologique dans un véhicule qui comprend un perfluorohydrocarbure présentant une pression de vapeur à 37°C comprise entre 133,3 et 2133,2 Pa (1 à 16 mm Hg), le véhicule étant présent dans une quantité de 98 à 99,99% en poids lorsque le médicament est un médicament oculaire et de 97 à 99,99% en poids lorsque le médicament est un médicament dermatologique, les deux pourcentages relativement à la solution, suspension ou mico-émulsion.